# EUROPÄISCHE PATENTSCHRIFT

(11) **EP 3 183 030 B1**
(45) Veröffentlichungstag und Bekanntmachung des Hinweises auf die Patenterteilung: **11.09.2019**
(21) Anmeldenummer: 15787235.9
(22) Anmeldetag: 30.10.2015
(51) Int. Cl.: A61N 1/36, A61N 5/06

(54) **VORRICHTUNG ZUR EFFEKTIVEN INVASIVEN DESYNCHRONISIERENDEN NEUROSTIMULATION**
DEVICE FOR EFFECTIVE INVASIVE DESYNCHRONIZING NEUROSTIMULATION
DISPOSITIF DE NEUROSTIMULATION DÉSYNCHRONISANTE EFFRACTIVE EFFICACE

(30) Priorität: 03.11.2014 DE 102014115994
(43) Veröffentlichungstag der Anmeldung: 28.06.2017
(73) Patentinhaber: Forschungszentrum Jülich GmbH, 52425 Jülich (DE)
(72) Erfinder: TASS, Peter Alexander, 83684 Tegernsee (DE); POPOVYCH, Oleksandr, 52355 Düren (DE); XENAKIS, Markos, 15121 Athens (GR)
(74) Vertreter: Vossius & Partner Patentanwälte Rechtsanwälte mbB
(86) Internationale Anmeldenummer: PCT/EP2015/075287
(87) Internationale Veröffentlichungsnummer: WO 2016/071234

(56) Entgegenhaltungen:
- US-A1- 2010 331 912
- US-A1- 2011 201 977
- US-A1- 2013 090 519
- US-A1- 2013 190 663

## Beschreibung

Die Erfindung betrifft eine Vorrichtung zur effektiven invasiven desynchronisierenden Neurostimulation.

Bei Patienten mit neurologischen oder psychiatrischen Erkrankungen, z. B. Morbus Parkinson, essentiellem Tremor, Epilepsie, Funktionsstörungen nach Schlaganfall, Dystonie oder Zwangserkrankungen, sind Nervenzellverbände in umschriebenen Bereichen des Gehirns krankhaft, z. B. übersteigert synchron, aktiv. In diesem Fall bildet eine große Anzahl von Neuronen synchron Aktionspotentiale aus, d. h., die beteiligten Neuronen feuern übermäßig synchron. Beim Gesunden hingegen feuern die Neuronen in diesen Hirngebieten qualitativ anders, z. B. auf unkorrelierte Weise.

Beim Morbus Parkinson verändert die pathologisch synchrone Aktivität die neuronale Aktivität in anderen Hirngebieten, z. B. in Arealen der Großhirnrinde wie dem primär motorischen Cortex. Dabei zwingt die pathologisch synchrone Aktivität im Bereich des Thalamus und der Basalgaglien beispielsweise den Großhirnrindenarealen ihren Rhythmus auf, so dass schließlich die von diesen Arealen gesteuerten Muskeln pathologische Aktivität, z. B. ein rhythmisches Zittern (Tremor), entfalten.

Zur Behandlung von medikamentös nicht hinreichend behandelbaren Parkinsonpatienten wird die tiefe Hirnstimulation eingesetzt. Hierbei werden Tiefenelektroden in speziellen Hirngebieten, z. B. im Nucleus subthalamicus, implantiert. Zur Linderung der Symptome wird über die Tiefenelektroden eine elektrische Reizung durchgeführt. Bei der Standard-Hochfrequenz-Stimulation zur Behandlung der Parkinson'schen Erkrankung wird eine sogenannte Hochfrequenz-Dauerreizung bei Frequenzen von über 100 Hz durchgeführt. Diese Behandlungsart hat keine lang anhaltenden therapeutischen Effekte (vgl. P. Temperli, J. Ghika, J.-G. Villemure, P. Burkhard, J. Bogousslavsky, and F. Vingerhoets: How do parkinsonian signs return after discontinuation of subthalamic DBS? Neurology 60, 78 (2003)). Mit weniger Reizstrom kommt die "Coordinated Reset"-Stimulation (CR-Stimulation) aus, die obendrein lang anhaltende therapeutische Effekte haben kann (P. A. Tass, L. Qin, C. Hauptmann, S. Doveros, E. Bezard, T. Boraud, W. G. Meissner: Coordinated reset neuromodulation has sustained after-effects in parkinsonian monkeys. Annals of Neurology 72, 816-820 (2012); I. Adamchic, C. Hauptmann, U. B. Barnikol, N. Pawelcyk, O.V. Popovych, T. Barnikol, A. Silchenko, J. Volkmann, G. Deuschl, W. Meissner, M. Maarouf, V. Sturm, H.-J. Freund, P. A. Tass: Coordinated Reset Has Lasting Aftereffects in Patients with Parkinson's Disease. Movement Disorders (online veröffentlicht, 2014)).

Bei anderen Erkrankungen, z. B. bei medikamentös nicht ausreichend behandelbaren Epilepsien, werden neben Tiefenelektroden auch andere, z. B. epikortikale oder epidurale Elektroden, implantiert. Bei weiteren Erkrankungen, z. B. chronischen Schmerz-Syndromen, ist es üblich, nicht nur mittels Tiefenelektroden im Gehirn, sondern auch mittels z. B. epiduralen Elektroden das Rückenmark zu reizen. Anders als die CR-Stimulation haben die meisten anderen Stimulationsarten keine lang anhaltenden therapeutischen Effekte.

Therapeutische Effekte können auch durch direkte Stimulation des Hirngewebes bzw. Rückenmarks mit Licht, z. B. über implantierte Lichtleiter, erzielt werden. Hierbei können auch unterschiedliche raum-zeitliche Stimulationsmuster, wie z. B. die CR-Stimulation, zur Anwendung kommen.

Bei der herkömmlichen Behandlung von Hirn- und Rückenmarkserkrankungen, bei der Elektroden oder Lichtleiter oder vergleichbare Stimulationseinheiten im Hirn und/oder Rückenmark des Patienten implantiert werden, um durch eine elektrische und/oder optische Reizung des Hirngewebes bzw. Rückenmarks therapeutische Effekte zu erzielen, kann es zu Limitationen kommen. Derartige Stimulationsbehandlungen können Nebenwirkungen hervorrufen, z. B. durch die nicht gewünschte Reizung von benachbarten Strukturen infolge der Ausbreitung von Reizströmen oder durch die aus anatomischen Gründen schwer vermeidbare gleichzeitige Reizung z. B. von Faserbahnen und/oder Fasern, welche in der Nachbarschaft des Zielgebiets oder sogar durch das Zielgebiet verlaufen. Derartige Situationen ergeben sich z. B. durch die charakteristische enge anatomische Nachbarschaft von dem bei der Elektrodenimplantation anvisierten Zielpunkt und anderen anatomischen Strukturen (deren Reizung zu Nebenwirkungen führt), durch spezielle individuelle anatomische Randbedingungen (z. B. im Sinne der Lage von Blutgefäßen, die bei der Implantation der Elektroden geschont werden müssen) oder auch durch suboptimale oder gar fehlerhafte Elektrodenimplantation.

Beispielsweise können durch ungünstig platzierte Elektroden Nebenwirkungen auftreten, die nur dann verschwinden, wenn die Stimulationsstärke so stark verringert wird, dass die gewünschten Wirkungen in nicht ausreichendem Maß bzw. überhaupt nicht mehr auftreten. Analog kann ein ungenügender Stimulationseffekt nicht durch ein beliebig starkes Anwachsen der Stimulationsintensität kompensiert werden, da es hierdurch typischerweise zu Nebenwirkungen oder sogar Schädigungen des Gewebes kommt. Gegebenenfalls müssen schlecht platzierte Elektroden oder sonstige Stimulationseinheiten neu implantiert werden, um sie so zu platzieren, dass die Behandlung effizient ist. Eine erneute Implantation ist immer mit einem Risiko verbunden, z. B. durch eine Verletzung von Gefäßen oder eine Infektion.

Eine herkömmliche Vorrichtung zur Unterdrückung einer krankhaft synchronen und oszillatorischen neuronalen Aktivität mit den Merkmalen des Oberbegriffs des Anspruchs 1 ist aus dem Dokument US 2011 / 0 201 977 A1 bekannt.

Der Erfindung liegt die Aufgabe zugrunde, eine Vorrichtung anzugeben, die es ermöglicht, durch Reizung mit minimalsten Reizstärken gute und insbesondere lang anhaltende therapeutische Effekte zu erzielen.

Die der Erfindung zugrunde liegende Aufgabenstellung wird durch die Merkmale des Anspruchs 1 gelöst. Vorteilhafte Weiterbildungen und Ausgestaltungen der Erfindung sind in den Unteransprüchen angegeben.

Die Erfindung wird nachfolgend in beispielhafter Weise unter Bezugnahme auf die Zeichnungen näher erläutert. In diesen zeigen:
- Fig. 1: eine schematische Darstellung einer Vorrichtung zur Unterdrückung einer krankhaft synchronen und oszillatorischen neuronalen Aktivität und insbesondere zur Desynchronisierung von Neuronen mit einer krankhaft synchronen und oszillatorischen Aktivität gemäß einer ersten Ausgestaltung;
- Fig. 2: eine schematische Darstellung einer Vorrichtung zur Unterdrückung einer krankhaft synchronen und oszillatorischen neuronalen Aktivität und insbesondere zur Desynchronisierung von Neuronen mit einer krankhaft synchronen und oszillatorischen Aktivität gemäß einer zweiten Ausgestaltung;
- Fig. 3: ein Flussdiagramm zur Veranschaulichung einer Regelung der Längen von Stimulationsphasen und Stimulationspausen gemäß einer ersten Variante;
- Fig. 4: ein Flussdiagramm zur Veranschaulichung einer Regelung der Längen von Stimulationsphasen und Stimulationspausen gemäß einer zweiten Variante;
- Fig. 5: eine schematische Darstellung einer Vorrichtung zur elektrischen Stimulation von Neuronen mit einer krankhaft synchronen und oszillatorischen neuronalen Aktivität;
- Fig. 6: eine schematische Darstellung einer CR-Reizfolge zur Stimulation einer Neuronenpopulation;
- Fig. 7: Graphen zur Veranschaulichung einer effektiven und einer ineffektiven CR-Stimulation;
- Fig. 8 bis 12: Graphen zur Veranschaulichung von CR-Stimulationen mit unterschiedlichen Stimulationsphasenlängen und Stimulationspausenlängen; und
- Fig. 13: ein Diagramm zur Darstellung der Effektivität von CR-Stimulationen mit unterschiedlichen Stimulationsphasenlängen und Stimulationspausenlängen.

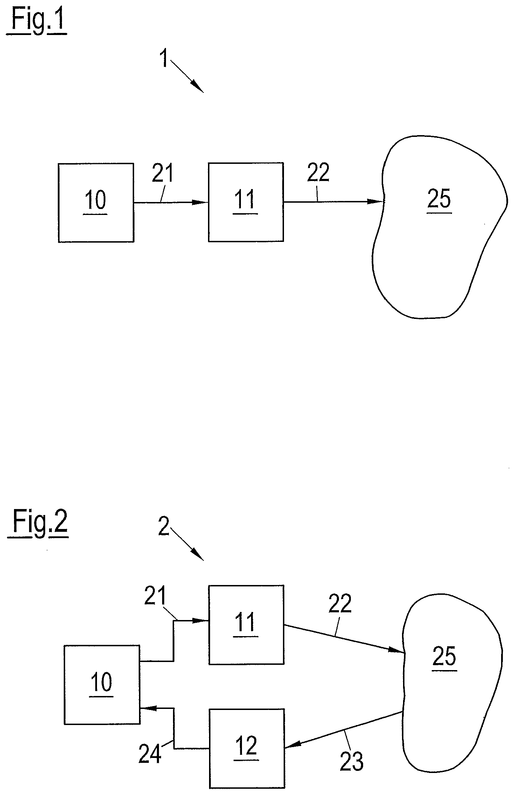

In Fig. 1 ist schematisch eine Vorrichtung 1 zur Stimulation von Neuronen mit einer krankhaft synchronen und oszillatorischen neuronalen Aktivität dargestellt. Die Vorrichtung 1 besteht aus einer Steuer- und Analyseeinheit 10 und einer Stimulationseinheit 11. Während des Betriebs der Vorrichtung 1 führt die Steuer- und Analyseeinheit 10 eine Steuerung der Stimulationseinheit 11 durch. Dazu erzeugt die Steuer- und Analyseeinheit 10 Steuersignale 21, die von der Stimulationseinheit 11 entgegengenommen werden.

Die Stimulationseinheit 11 wird operativ in den Körper des Patienten implantiert und erzeugt anhand der Steuersignale 21 elektrische und/oder optische Reize 22, die dem Hirn und/oder Rückenmark 25 des Patienten verabreicht werden. Die Reize 22 sind dazu ausgelegt, bei einer Verabreichung an den Patienten die krankhaft synchrone und oszillatorische neuronale Aktivität zu unterdrücken und insbesondere die Neuronen mit der krankhaft synchronen und oszillatorischen Aktivität zu desynchronisieren.

Die Steuer- und Analyseeinheit 10 kann eine nicht-invasive Einheit sein, d. h., während des Betriebs der Vorrichtung 1 befindet sie sich außerhalb des Körpers des Patienten und wird nicht operativ in den Körper des Patienten implantiert.

Während des Betriebs der Vorrichtung 1 kann, falls ein ungenügender Stimulationseffekt festgestellt wird, die Effizienz der Stimulation durch die Einfügung von Stimulationspausen, beispielsweise durch den Arzt oder Anwender, bei geringen Reizstärken verbessert werden. Während der Stimulationspausen findet keine Applikation von Reizen statt, welche die krankhaft synchrone und oszillatorische neuronale Aktivität unterdrücken könnten. Es ist jedoch denkbar, dass andere Reize, die nicht dazu ausgelegt sind, die krankhaft synchrone und oszillatorische neuronale Aktivität zu unterdrücken, während der Stimulationspausen insbesondere mit der Stimulationseinheit 11 appliziert werden. Gemäß einer weiteren Ausführungsform wird während der Stimulationspausen auf Stimulation jeglicher Art mit Hilfe der Stimulationseinheit 11 verzichtet. Weiterhin können z. B. im Fall von Nebenwirkungen und/oder ungünstig platzierten Elektroden die vorstehend beschriebenen Stimulationspausen eingefügt werden, um eine effiziente Stimulation bei geringen Reizstärken zu ermöglichen. Die Dauer der Stimulationspausen kann konstant gehalten, durch den Arzt oder Anwender eingestellt oder wie weiter unten beschrieben geregelt werden.

Die Erfindung nutzt einen kontraintuitiven Zusammenhang: Je geringer der durch die schwache Stimulation erzielte Erfolg ist, desto längere Pausen werden in den Stimulationsvorgang eingeschoben. Insbesondere kann durch das Einschieben von derartigen Pausen bewirkt werden, dass eine ansonsten wirkungslose Stimulation effektiv ist.

Der der Erfindung zugrunde liegende kontraintuitive Mechanismus lässt sich durch die folgenden Überlegungen plausibel machen. Infolge der synaptischen Plastizität sind Verbände von Neuronen sehr plastisch, d. h., sie können in einer Vielzahl von unterschiedlichen stabilen Zuständen vorliegen. Z. B. in Zuständen mit niedriger mittlerer synaptischer Verbindungsstärke und unsynchroner neuronaler Aktivität, d. h., die Neuronen feuern auf unkorrelierte Weise, oder in Zuständen mit stark ausgeprägter mittlerer synaptischer Verbindungsstärke und synchroner neuronaler Aktivität, d. h., die Neuronen feuern auf korrelierte Weise, z. B. im Takt, also koinzident. Zwischen diesen beiden Extremen gibt es typischerweise eine Vielzahl stabiler Zustände mit intermediärer mittlerer synaptischer Verbindungsstärke und intermediärer Ausprägung der neuronalen Synchronisation. Es liegt also im mathematischen Sinn eine Multistabilität vor. Die Erfindung nutzt die überraschende Tatsache aus, dass das System, d. h. die stimulierte Neuronenpopulation, selbst mit schwacher Stimulation von einem Attraktor (stabilen Zustand) zum nächsten geschoben werden kann, wenn zwischen den Stimulationsphasen eine hinreichend lange Pause ist, während der das System in den neuen Attraktor spontan (d.h. ohne Stimulation) hineingezogen wird, was unter Stimulation nicht möglich wäre. Durch die portionierte Stimulation gelangt das System quasi schrittweise von stark synchronen Attraktoren zu zunehmend schwächer synchronen Attraktoren.

Das Einfügen hinreichend langer Stimulationspausen ermöglicht eine effiziente Stimulation bei geringen Reizstärken. Die Reizstärke kann dann bis zu einem Faktor 2 bis 3 mal geringer sein als die Mindestreizstärke, welche bei dauerhafter Stimulation, d. h. bei einer Stimulation ohne die hierin beschriebenen Stimulationspausen, zu einer lang anhaltenden Desynchronisation führt. Insbesondere kann die Reizstärke der erfindungsgemäßen Stimulation mit Stimulationspausen in einem Bereich von 1/3 der Mindestreizstärke bis zu 2/3 der Mindestreizstärke, welche bei einer dauerhaften Stimulation ohne die erfindungsgemäßen Stimulationspausen zu einer lang anhaltenden Desynchronisation führt, liegen.

Die Länge einer Stimulationspause zwischen zwei aufeinander folgenden Stimulationsabschnitten kann mindestens 3 Minuten betragen, kann aber auch wesentlich länger sein und beispielsweise mindestens 5 Minuten oder mindestens 10 Minuten oder mindestens 20 Minuten oder mindestens 30 Minuten oder mindestens 1 Stunde oder mindestens 2 Stunden oder mindestens 3 Stunden betragen. Um erste Effekte zu erzielen, muss die Stimulationspausenlänge mindestens ca. 200 Perioden der zu desynchronisierenden Oszillation entsprechen. Eine ausgeprägte Desynchronisation lässt sich erst ab ca. 1.000 bis sogar 22.000 Perioden erzielen. Im Falle einer Delta-Oszillation mit einer Frequenz im Bereich von 1 bis 4 Hz beträgt die Periodenlänge z. B. 500 ms bei 2 Hz. D. h., gute Effekte ergeben sich bei Pausen im Minuten- oder sogar Stundenbereich (1.000 bzw. 22.000 Perioden entsprechen dann ca. 8.3 min bzw. 3 Stunden). Die Periode der pathologischen Oszillation kann beispielsweise am Patienten gemessen werden, es können aber auch Literatur- oder Erfahrungswerte verwendet werden.

Weiterhin kann vorzugsweise neben der Länge der Stimulationspausen auch die Länge der Stimulationsphasen, in denen eine Stimulation stattfindet, eingestellt werden, um bei geringen Reizstärken die Effizienz der Stimulation zu verbessern. Die Länge der Stimulationsphasen kann in gleicher Weise wie die Länge der Stimulationspausen konstant gehalten, durch den Arzt oder Anwender eingestellt oder wie weiter unten beschrieben geregelt werden.

Vorzugsweise können die Stimulationspausen bei zu geringem Stimulationseffekt verlängert und die Stimulationsphasen ebenfalls verlängert werden.

Beispielsweise können die Stimulationspausen und Stimulationsphasen jeweils gleich lang sein und somit gleichermaßen anwachsen. Weiterhin können die Stimulationsphasen auch zu Beginn kürzer als die Stimulationspausen sein und bei zu geringem Stimulationseffekt überproportional wachsen. Ferner kann jede geeignete andere Relation zwischen der Dauer der Stimulationspausen und der Dauer der Stimulationsphasen eingestellt werden.

In Fig. 2 ist schematisch eine Vorrichtung 2 zur Stimulation von Neuronen mit einer krankhaft synchronen und oszillatorischen neuronalen Aktivität dargestellt. Die Vorrichtung 2 stellt eine Weiterbildung der in Fig. 1 dargestellten Vorrichtung 1 dar. Die Vorrichtung 2 weist genauso wie die Vorrichtung 1 eine Steuer- und Analyseeinheit 10 und eine Stimulationseinheit 11 auf. Während des Betriebs der Vorrichtung 1 führt die Steuer- und Analyseeinheit 10 eine Steuerung der Stimulationseinheit 11 durch. Dazu erzeugt die Steuer- und Analyseeinheit 10 Steuersignale 21, die von der Stimulationseinheit 11 entgegengenommen werden.

Wie oben beschrieben wird die Stimulationseinheit 11 operativ in den Körper des Patienten implantiert und erzeugt anhand der Steuersignale 21 elektrische und/oder optische Reize 22, die dem Hirn und/oder Rückenmark 25 des Patienten verabreicht werden.

Darüber hinaus umfasst die Vorrichtung 2 eine Messeinheit 12. Der durch die Reize 22 erzielte Stimulationseffekt wird mit Hilfe der Messeinheit 12 überwacht. Die Messeinheit 12 nimmt ein oder mehrere am Patienten gemessene Messsignale 23 auf, wandelt diese gegebenenfalls in elektrische Signale 24 um und führt sie der Steuer- und Analyseeinheit 10 zu. Insbesondere kann mittels der Messeinheit 12 die neuronale Aktivität in dem stimulierten Zielgebiet oder einem mit dem Zielgebiet verbundenen Gebiet gemessen werden, wobei die neuronale Aktivität dieses Gebiets mit der neuronalen Aktivität des Zielgebiets hinreichend eng korreliert. Mittels der Messeinheit 12 kann auch eine nicht-neuronale, z. B. muskuläre Aktivität oder die Aktivierung des autonomen Nervensystems, gemessen werden, sofern diese mit der neuronalen Aktivität des Zielgebiets hinreichend eng korreliert sind.

Die Messeinheit 12 enthält ein oder mehrere Sensoren, die es insbesondere ermöglichen, eine Ab- bzw. Zunahme der Amplitude der pathologischen oszillatorischen Aktivität nachzuweisen.

Als Sensoren können nicht-invasive Sensoren eingesetzt werden, z. B. Elektroenzephalographie (EEG)-Elektroden, Magnetenzephalographie (MEG)-Sensoren und Sensoren zur Messung lokaler Feldpotentiale (LFP). Die neuronale Aktivität kann auch indirekt durch Messung der damit einhergehenden Muskelaktivität mittels Elektromyographie (EMG) oder indirekt durch Messung der Aktivierung des autonomen Nervensystems mittels Messung des Hautleitwiderstands ermittelt werden.

Alternativ können die Sensoren in den Körper des Patienten implantiert sein. Als invasive Sensoren können beispielsweise epikortikale Elektroden, Tiefenhirnelektroden zur Messung von z. B. lokalen Feldpotentialen, sub- oder epidurale Hirnelektroden, subkutane EEG-Elektroden und sub- oder epidurale Rückenmarkselektroden dienen. Die Tiefenelektroden zur Messung der lokalen Feldpotentiale können auch baulich vereint oder sogar identisch mit den für die Stimulation verwendeten Tiefenelektroden sein.

Die Steuer- und Analyseeinheit 10 verarbeitet die Signale 24, z. B. können die Signale 24 verstärkt und/oder gefiltert werden, und analysiert die verarbeiteten Signale 24. Anhand der Ergebnisse dieser Analyse steuert die Steuer- und Analyseeinheit 10 insbesondere die Stimulationseinheit 11 an. Zur Durchführung ihrer Aufgaben kann die Steuer- und Analyseeinheit 10 z. B. einen Prozessor (z. B. einen Mikrocontroller) enthalten. Die Steuer- und Analyseeinheit 10 überprüft anhand der in Reaktion auf die Applikation der Reize aufgenommenen Messsignale den Stimulationserfolg und stellt die Stimulationsparameter, insbesondere die Längen der oben im Zusammenhang mit Fig. 1 beschriebenen Stimulationspausen, in Abhängigkeit vom Stimulationserfolg ein. Im Fall von Nebenwirkungen und/oder ungünstig platzierten Elektroden und/oder generell bei ungenügendem Stimulationseffekt kann im Betrieb bei geringen Reizstärken die Effizienz der Stimulation durch die Anpassung der Stimulationspausen verbessert werden. Die Dauer der Stimulationspausen und die Dauer der Stimulationsphasen kann bei zu geringem Stimulationseffekt so geregelt werden, dass sich wieder ein Stimulationseffekt einstellt.

Der Stimulationserfolg kann insbesondere mittels eines Schwellwertvergleichs überprüft werden. Je nachdem welche Signale zur Ermittlung des Stimulationserfolgs herangezogen werden, ergeben sich unterschiedliche Schwellwertvergleiche. Wird z. B. die krankhafte neuronale Synchronisation über die Sensoren der Messeinheit 12, z. B. EEG-Elektroden oder Tiefelektroden (als LFP-Signal), gemessen, reicht erfahrungsgemäß die Absenkung der Synchronisation um z. B. mindestens 20 % im Vergleich zur Situation ohne Stimulation, um einen ausreichenden Stimulationserfolg festzustellen. Gemäß einer Ausgestaltung kann ein nicht ausreichender Stimulationserfolg festgestellt werden, wenn sich die krankhafte neuronale Synchronisation durch die Applikation der Reize 22 nicht um mindestens einen vorgegebenen Wert verringert. Falls Symptome des Patienten zur Ermittlung des Stimulationserfolgs herangezogen werden, hängt es von der Art der verwandten klinischen Parameter ab, welche Abnahme als klinisch relevante Besserung anzusehen ist. Derartige Abnahmewerte (z. B. im Sinne der sog. minimalen klinisch wahrnehmbaren Verbesserung) sind dem Fachmann bekannt.

Es können in abwechselnder Reihenfolge Stimulationsphasen, in denen das Hirn und/oder Rückenmark 25 des Patienten mit den von der Stimulationseinheit 11 erzeugten Reizen 22 stimuliert wird, und Stimulationspausen, in denen keine Reize 22 appliziert werden, eingehalten werden.

Für die Regelung der Dauer L_{Stim} der Stimulationsphasen und der Dauer L_{Pause} der Stimulationspausen können z. B. Standardverfahren der Zweigrößenregelung verwandt werden. Es kann aber auch medizinisches a priori-Wissen verwandt werden, wobei die Längen L_{Stim} und L_{Pause} von einem Startwert in der (L_{Stim}, L_{Pause})-Ebene aus mit konstanter oder sukzessiv anwachsender oder deterministisch und/oder chaotisch variierter Schrittweite entlang einer Geraden oder gebogenen Kurve vergrößert werden. Beispielsweise kann das Verhältnis L_{Stim}/L_{Pause} im Rahmen dieses Regelungsvorgangs von 1 / n auf n anwachsen, wobei n beispielsweise eine Zahl im Bereich von 2 bis 10 ist, z. B. 3 oder 4 oder 5.

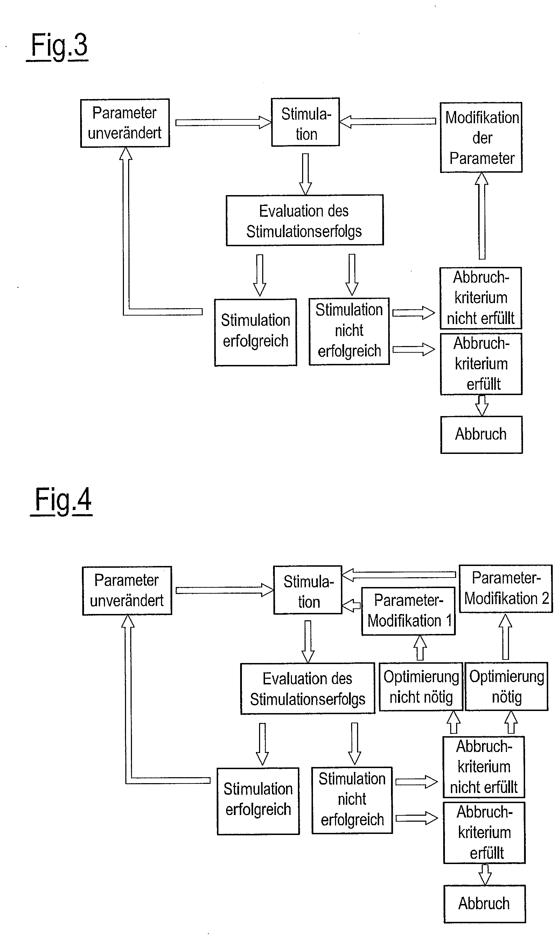

Fig. 3 zeigt ein Flussdiagramm für eine beispielhafte Regelung der Längen L_{Stim} und L_{Pause} der Stimulationsphasen und -pausen gemäß einer ersten Variante. Die Längen L_{Stim} und L_{Pause} können beispielsweise während des gesamten Vorgangs gleich groß sein, d. h., es gilt L_{Stim} = L_{Pause} = A. Als Stellgröße für das Regelungsverfahren kann aber auch das Verhältnis L_{Stim}/L_{Pause} herangezogen werden. In letzterem Fall kann die Länge L_{Stim} beispielsweise um bis zu ±5 % oder bis zu ±10 % oder bis zu ±25 % von der Länge L_{Pause} abweichen, d. h., es gilt L_{Stim} = (1+ε)L_{Pause} = A, wobei ε in den vorstehenden genannten Fällen bis zu ±0,05 bzw. ±0,1 bzw. ±0,25 beträgt.

Der Parameter A wird von einem voreingestellten Startwert aus so lange konstant gehalten, bis die Steuer- und Analyseeinheit 10 die Stimulation als nicht erfolgreich klassifiziert. Dann wird der Parameter A insbesondere schrittweise vergrößert, bis die Steuer- und Analyseeinheit 10 anhand der von der Messeinheit 12 aufgenommenen Messsignale 24 feststellt, dass die Stimulation wieder hinreichend erfolgreich ist.

Gemäß einer Ausgestaltung wird der Parameter A bei nicht ausreichendem Stimulationserfolg so lange insbesondere schrittweise vergrößert, bis ein hinreichender Stimulationserfolg festgestellt wird oder ein Abbruchkriterium erfüllt ist. Das Abbruchkriterium soll ermitteln, wann trotz hinreichend großem und hinreichend vertretbarem Aufwand kein ausreichender Stimulationserfolg zu erwarten ist.

Das Abbruchkriterium kann z. B. erfüllt sein, wenn mindestens eines von mehreren Kriterien erfüllt ist. Ein Abbruchkriterium K1 kann z. B. erfüllt sein, wenn eine vorgegebene Behandlungsdauer, z. B. von 12 Wochen, überschritten wird, und ist ansonsten nicht erfüllt. Die Wahl von der vorgegebenen Behandlungsdauer hängt vom jeweiligen Krankheitsbild bzw. - stadium ab und spiegelt die klinische Erfahrung wider.

Fig. 4 zeigt ein Flussdiagramm für eine weitere beispielhafte Regelung der Längen L_{Stim} und L_{Pause} der Stimulationsphasen und -pausen gemäß einer zweiten Variante. Die in Fig. 4 dargestellte Regelung ist in vielen Teilen identisch mit der Regelung aus Fig. 3, ist aber in folgender Hinsicht komplexer. Empirisch hat sich gezeigt, dass die in Fig. 3 gezeigte Regelung robust funktioniert. Es kann aber typischerweise deutlich an Zeit gespart werden, wenn - wie oben beschrieben - das Verhältnis L_{Stim}/L_{Pause} im Rahmen dieses Regelungsvorgangs von 1/n auf n angepasst an den Stimulationserfolg anwächst, wobei n beispielsweise eine Zahl im Bereich von 2 und 10 ist, z. B. 3 oder 4 oder 5. Sollte diese Anpassungsregel nicht hinreichend funktionieren, ist das Kriterium "Optimierung nötig" erfüllt. In diesem Falle wechselt die erfindungsgemäße Vorrichtung zu dem in Fig. 3 dargestelltem noch robusteren Regel-Verfahren. Konkret ist das Kriterium "Optimierung nötig" analog zu einem Abbruchkriterium, d. h., es stellt sich in einer bestimmten Zeit bzw. nach einer bestimmten Anzahl von Regelschritten kein hinreichend stark ausgeprägter therapeutischer Erfolg ein.

Gemäß einer Ausgestaltung ist ein Optimierungskriterium K2 erfüllt, wenn mittels invasiver und/oder nicht-invasiver Sensoren gemessene Biomarker und/oder Selbstbeurteilungsskalen, z. B. Befindlichkeitsskalen oder Lebensqualitätsskalen, die z .B. über ein mobiles Gerät (wie ein iPhone) eingegeben und entsprechend evaluiert werden, sich nicht hinreichend verbessern. Für die hier verwendeten invasiven und/oder nicht invasiven Sensoren können die Sensoren der Messeinheit 12 verwendet werden. Insbesondere können unterschiedliche Formen von Elektroden, z. B. Tiefenelektroden oder epikortikale Elektroden, als invasive Sensoren eingesetzt werden. Als nicht-invasive Sensoren kommen z. B. chronisch oder intermittent genutzte EEG-Elektroden oder Akzelerometer zur Detektion charakteristischer Bewegungsmuster, wie z. B. Tremor, Akinese oder epileptische Anfälle, infrage. Ein Biomarker ist z. B. die spektrale Dichte in einem charakteristischen, dem Fachmann bekannten Frequenzbereich (z. B. dem von ca. 8 bis 30 Hz verlaufenden Beta-Band beim Parkinsonpatienten) des über Tiefenelektroden abgeleiteten lokalen Feldpotentials.

Wenn der oder die Biomarker bzw. die Selbstbeurteilungsskalen (i) nicht innerhalb einer vorgegebenen Zeit, z. B. 4 Wochen, um einen bestimmten Prozentsatz vom Ausgangswert, z. B. 20 % oder 50 % im mehrtägigen Mittel je nach Erkrankung bzw. Krankheitsstadium, abnehmen und/oder (ii) nicht nach dem m-ten Anpassungsschritt des Parameters A, z. B. m = 3, um diesen Prozentsatz vom Ausgangswert abgenommen haben, ist gemäß einer Ausgestaltung das Kriterium K2 erfüllt. Ansonsten ist das Kriterium K2 nicht erfüllt. Hierbei sind die Anpassungsschritte von A konstant oder sukzessiv anwachsend oder werden deterministisch und/oder chaotisch variiert. Die Wiederholungszahl m entspricht der klinischen Erfahrung, d. h. der Zeitskala, auf der der therapeutische Erfolg sich bei der jeweiligen Erkrankung einstellen kann.

Anstelle der in den Fig. 3 und 4 dargestellten Regelungen kann auch eine Stimulation durchgeführt werden, bei der die Längen L_{Stim} der Stimulationsphasen und die Längen L_{Pause} der Stimulationspausen konstant sind, d. h., L_{Stim} = L_{Pause} = A oder L_{Stim} = (1+ε)L_{Pause} = A mit ε im Bereich von ±0,05, ±0,1 oder ±0,25, und die Stimulation mit konstantem A so lange durchgeführt wird, bis die Stimulation von der Steuer- und Analyseeinheit 10 als nicht erfolgreich klassifiziert wird, d. h., bis ein wie im Zusammenhang mit der Regelung nach Fig. 3 beschriebenes Abbruchkriterium erfüllt ist. Die Stimulation wird dann beendet. Die Vorrichtung 2 kann dann eine entsprechende Meldung ("Stimulation abgebrochen") an den Patienten, z. B. auf einem Display oder durch ein blinkendes Kontrolllämpchen oder dergleichen, erbringen. Diese Meldung kann auch per Funk, z. B. als SMS, E-Mail oder dergleichen, an den Arzt gehen. Als Alternative hierzu kann bei Erreichen des Abbruchkriteriums die Behandlung auch weiter durchgeführt werden, die entsprechende Meldung an den Patienten lautet dann z. B. "Bitte Arzt aufsuchen", und die SMS/E-Mail geht an den behandelnden Arzt, um ihn über die insuffiziente Therapie zu informieren.

Die invasive Stimulation, z. B. invasive CR-Stimulation, kann eine "open loop"-Stimulation oder eine "closed loop"-Stimulation sein. Im Fall der "closed loop"-Stimulation werden implantierte Sensoren der Messeinheit 12, z. B. Tiefenelektroden, epikortikale Elektroden, und/oder nicht implantierte Sensoren der Messeinheit 12, z. B. chronisch oder intermittent genutzte EEG-Elektroden oder Akzelerometer zur Detektion charakteristischer Bewegungsmuster (Tremor, Akinese, epileptische Anfälle) oder Elektroden zur Messung des Hautleitwiderstands verwandt, um die Stimulation zu steuern. Diese Sensoren können auch verwandt werden, (i) um die Regelung der Stimulationsphasen und Stimulationspausen, d. h. deren Anpassung an den therapeutischen Effekt, zu realisieren bzw. (ii) um Optimierungs- bzw. Abbruchkriterien wie oben in Zusammenhang mit den Fig. 3 und 4 beschrieben zu ermitteln. Es können aber auch für die Regelung der Längen L_{Stim} und L_{Pause} der Stimulationsphasen und -pausen andere Sensoren bzw. andere Signale bzw. andere Signalbestandteile bzw. andere (ggfs. aus denselben Ausgangssignalen ermittelte) dynamische Biomarker für die "closed loop"-Stimulation verwandt werden.

Die einzelnen Komponenten der Vorrichtungen 1 und 2, insbesondere die Steuer- und Analyseeinheit 10, die Stimulationseinheit 11 und/oder die Messeinheit 12, können baulich voneinander getrennt sein. Die Vorrichtungen 1 und 2 können daher auch als Systeme aufgefasst werden.

Die Stimulationseinheit 11 kann z. B. ein Hirnschrittmacher sein und weist in diesem Fall eine oder mehrere implantierbare Elektroden, z. B. Tiefenelektroden, sowie gegebenenfalls dazwischen geschaltete Verbindungskabel auf. Die Elektroden der Stimulationseinheit 11 bestehen typischerweise aus einem isolierten Elektrodenschaft und einer Mehrzahl von Stimulationskontaktflächen, die in den Elektrodenschaft eingebracht worden sind.

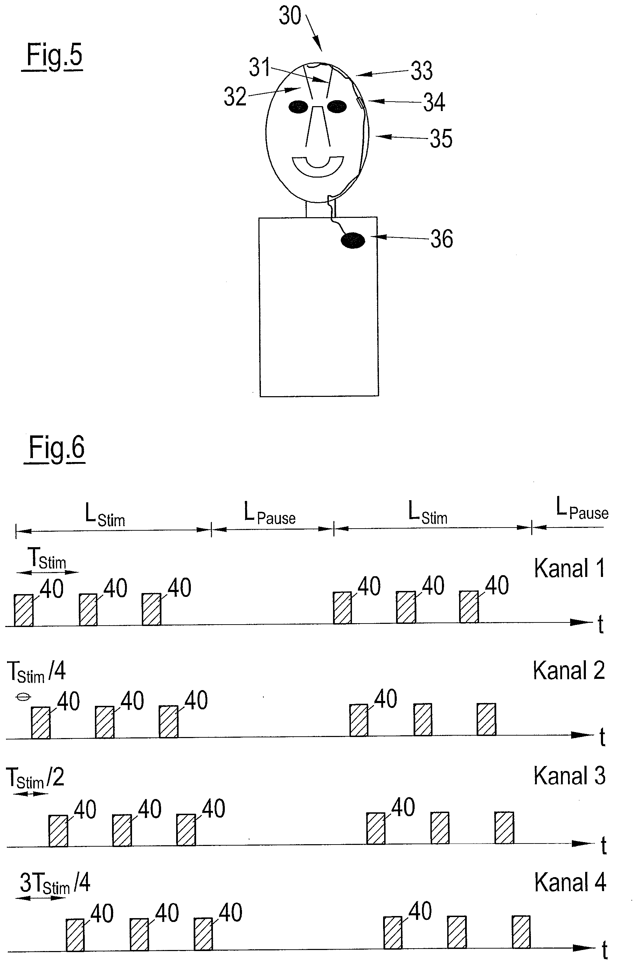

Fig. 5 zeigt schematisch eine Vorrichtung 30 zur invasiven elektrischen Stimulation von Neuronen mit einer krankhaft synchronen und oszillatorischen neuronalen Aktivität gemäß einer Ausführungsform der Erfindung. Die Vorrichtung 30 umfasst zwei Elektroden 31, 32, die in das Hirn des Patienten implantiert sind und über Kabel 33 mit einem Konnektor 34 verbunden sind. Der Konnektor 34 wiederum ist über ein Kabel 35 mit einer Steuer- und Analyseeinheit 36 verbunden. Die Vorrichtung 30 kann die Funktionen der oben beschriebenen Vorrichtungen 1 und 2 aufweisen.

Implantierbare Stimulationseinheiten für die optische Stimulation von neuronalem Gewebe sind bekannt. Beispielsweise kann eine Lichtquelle, wie z. B. ein Laser, eine Laserdiode oder eine LED, einen Lichtstrahl erzeugen, der mit Hilfe einer Lichteinkopplung auf die Eingänge eines aus mehreren Lichtleitern bestehenden Faserbündels verteilt wird. Eine Steuereinheit gibt dabei z. B. vor, zu welchem Zeitpunkt ein einzelner Lichtpuls oder ein Zug von Lichtpulsen in welche Faser des Faserbündels eingekoppelt wird. Die Auskopplungspunkte der einzelnen Fasern des Fasernbündels, d. h. die Enden der Fasern, liegen an verschiedenen Stellen im Zielgebiet im Hirn oder Rückenmark des Patienten. Das Licht stimuliert somit unterschiedliche Orte des Zielgebiets in einer durch die Steuereinheit vorgegebenen zeitlichen Abfolge. Es können allerdings auch andere implantierbare Stimulationseinheiten verwendet werden, die sich zur direkten optischen Stimulation von neuronalem Gewebe eignen.

Die hierin beschriebenen Vorrichtungen, insbesondere die Vorrichtungen 1 und 2, können insbesondere zur Behandlung von neurologischen oder psychiatrischen Erkrankungen eingesetzt werden, z. B. Morbus Parkinson, essentiellem Tremor, Tremor infolge von Multipler Sklerose sowie anderen pathologischen Tremores, Dystonie, Epilepsie, Depression, Bewegungsstörungen, Kleinhirnerkrankungen, Zwangserkrankungen, Demenzerkrankungen, Morbus Alzheimer, Tourette-Syndrom, Autismus, Funktionsstörungen nach Schlaganfall, Spastik, Tinnitus, Schlafstörungen, Schizophrenie, Reizdarm-Syndrom, Suchterkrankungen, Borderline-Persönlichkeitsstörung, Aufmerksamkeits-Defizit-Syndrom, Aufmerksamkeits-Defizit-Hyperaktivitäts-Syndrom, Spielsucht, Neurosen, Fresssucht, Magersucht, Essstörungen, Burnout-Syndrom, Fibromyalgie, Migräne, Cluster-Kopfschmerz, allgemeiner Kopfschmerz, Neuralgie, Ataxie, Tic-Störung oder Hypertonie, sowie weiteren Erkrankungen, die durch krankhaft gesteigerte neuronale Synchronisation gekennzeichnet sind.

Die vorstehend genannten Krankheiten können durch eine Störung der bioelektrischen Kommunikation von Neuronenverbänden, die in spezifischen Schaltkreisen zusammengeschlossen sind, verursacht werden. Hierbei generiert eine Neuronenpopulation anhaltend krankhafte neuronale Aktivität und möglicherweise eine damit verbundene krankhafte Konnektivität (Netzwerkstruktur). Dabei bilden eine große Anzahl von Neuronen synchron Aktionspotentiale aus, d. h., die beteiligten Neuronen feuern übermäßig synchron. Hinzu kommt, dass die kranke Neuronenpopulation eine oszillatorische neuronale Aktivität aufweist, d. h., die Neuronen feuern rhythmisch. Im Fall von neurologischen oder psychiatrischen Erkrankungen liegt die mittlere Frequenz der krankhaften rhythmischen Aktivität der betroffenen Neuronenverbände etwa im Bereich von 1 bis 50 Hz, kann aber auch außerhalb dieses Bereichs liegen. Bei gesunden Menschen feuern die Neuronen hingegen qualitativ anders, z. B. auf unkorrelierte Weise.

Bei der oben erwähnten CR-Stimulation wird eine Neuronenpopulation im Hirn und/oder Rückenmark, die eine krankhaft synchrone und oszillatorische neuronale Aktivität aufweist, mit den elektrischen und/oder optischen Reizen entweder direkt stimuliert oder aber die Reize werden über das Nervensystem an die krankhaft aktive Neuronenpopulation weitergeleitet. Die Reize sind so ausgestaltet, dass die krankhaft synchrone Aktivität der Neuronenpopulation desynchronisiert wird. Eine durch die Stimulation bewirkte Senkung der Koinzidenzrate der Neuronen kann zu einer Senkung der synaptischen Gewichte und somit zu einem Verlernen der Tendenz zur Produktion krankhaft synchroner Aktivität führen.

Die bei der CR-Stimulation verabreichten Reize bewirken in der Neuronenpopulation ein Zurücksetzen, einen sogenannten Reset, der Phase der neuronalen Aktivität der stimulierten Neuronen. Durch das Zurücksetzen wird die Phase der stimulierten Neuronen unabhängig von dem aktuellen Phasenwert auf einen oder nahe zu einem bestimmten Phasenwert, z. B. 0°, gesetzt (in der Praxis ist es nicht möglich, einen bestimmten Phasenwert exakt einzustellen, dies ist für eine erfolgreiche CR-Stimulation aber auch nicht erforderlich). Somit wird die Phase der neuronalen Aktivität der krankhaften Neuronenpopulation mittels einer gezielten Stimulation kontrolliert. Ferner wird die krankhafte Neuronenpopulation mittels mehrerer Stimulationskontakte der Stimulationseinheit an unterschiedlichen Stellen stimuliert, so dass die Phase der neuronalen Aktivität der krankhaften Neuronenpopulation an den unterschiedlichen Stimulationsstellen zu unterschiedlichen Zeitpunkten zurückgesetzt werden kann. Im Ergebnis wird dadurch die krankhafte Neuronenpopulation, deren Neuronen zuvor synchron und mit gleicher Frequenz und Phase aktiv waren, in mehrere Subpopulationen aufgespalten. Innerhalb jeder der Subpopulationen sind die Neuronen nach dem Zurücksetzen der Phase weiterhin synchron und feuern auch weiterhin mit derselben pathologischen Frequenz, aber jede der Subpopulationen weist bezüglich ihrer neuronalen Aktivität die Phase auf, die ihr durch den von dem jeweiligen Stimulationskontakt generierten Reiz aufgezwungen wurde. Dies bedeutet, dass die neuronalen Aktivitäten der einzelnen Subpopulationen nach dem Zurücksetzen ihrer Phasen weiterhin einen in etwa sinusförmigen Verlauf mit derselben pathologischen Frequenz haben, aber unterschiedliche Phasen.

Bedingt durch die krankhafte Interaktion zwischen den Neuronen ist der durch die Stimulation erzeugte Zustand mit mindestens zwei Subpopulationen instabil, und die gesamte Neuronenpopulation nähert sich schnell einem Zustand kompletter Desynchronisation, in welchem die Neuronen unkorreliert feuern. Der gewünschte Zustand, d. h. die komplette Desynchronisation, ist somit nach der zeitversetzten (oder phasenverschobenen) Applikation der phasenrücksetzenden Reize nicht sofort vorhanden, sondern stellt sich meist innerhalb weniger Perioden oder gar in weniger als einer Periode der pathologischen Frequenz ein.

Fig. 6 zeigt ein Beispiel einer CR-Stimulation mit insgesamt vier Kanälen. Über jeden der vier Kanäle wird eine Subpopulation der krankhaften Neuronenpopulation stimuliert. In jedem der vier Kanäle werden elektrische und/oder optische Reize 40 in einer Sequenz periodisch mit der Periode Tstim appliziert, wobei die Periode Tstim nahe bei der mittleren Periode der pathologischen Oszillation der Neuronenpopulation liegt bzw. um bis zu ± 5%, ± 10% oder ± 20% von dem Literaturwert abweicht (typischerweise liegt f_{Stim} = 1/T_{Stim} im Bereich von 1 bis 30 Hz). Die Reize 40 bewirken eine Phasenrücksetzung der neuronalen Aktivität der jeweils stimulierten Subpopulation. Ferner beträgt die zeitliche Verzögerung zwischen den Sequenzen benachbarter Kanäle T_{Stim}/4, da vier Kanäle vorhanden sind. Für den allgemeinen Fall von N Kanälen würde die zeitliche Verzögerung benachbarter Kanäle T_{Stim}/N betragen (von diesem Wert kann auch um z. B. bis zu ± 5%, ± 10% oder ± 20% abgewichen werden). Ferner muss die Reihenfolge der Reizverabreichung über die N Kanäle nicht in jedem Stimulationszyklus identisch sein, sondern kann z. B. auch von Stimulationszyklus zu Stimulationszyklus randomisiert variiert werden.

Ferner sind in Fig. 6 die Längen L_{Stim} der Stimulationsphasen und die Längen L_{Pause} der Stimulationspausen dargestellt, die wie oben beschrieben eingestellt oder geregelt werden können. Zu beachten ist, dass in Fig. 6 die Längen L_{Stim} und L_{Pause} und die Längen der phasenrücksetzenden Reize 40 nicht maßstabsgetreu wiedergegeben sind.

Es sei darauf hingewiesen, dass auch bei herkömmlichen Stimulationsverfahren Pausen eingehalten werden können, in denen keine Reize appliziert werden. Beispielsweise kann bei der CR-Stimulation für n Zyklen stimuliert und für die folgenden m Zyklen nicht stimuliert werden und dieses Stimulationsmuster periodisch fortgeführt werden, wobei n und m kleine ganze Zahlen sind. Derartige Pausen können erfindungsgemäß auch während der Stimulationsphasen der Länge Lₛₜᵢₘ eingehalten werden. Die erfindungsgemäßen Stimulationspausen der Länge L_{Pause} unterscheiden sich jedoch von den Pausen während der Stimulationsphasen darin, dass sie nur dann eingehalten werden, wenn zuvor festgestellt wurde, dass der durch die Stimulation erzielte Stimulationserfolg nicht ausreichend ist, und/oder Nebenwirkungen auftreten und/oder die Stimulationseinheit im Körper des Patienten ungünstig platziert ist.

Anstelle der CR-Stimulation können auch anderen Stimulationsformen verwendet werden, sofern sich mit diesen Stimulationsformen bei der Desynchronisation von krankhaft aktiven Neuronenpopulationen lang anhaltende therapeutische Effekte erzielen lassen.

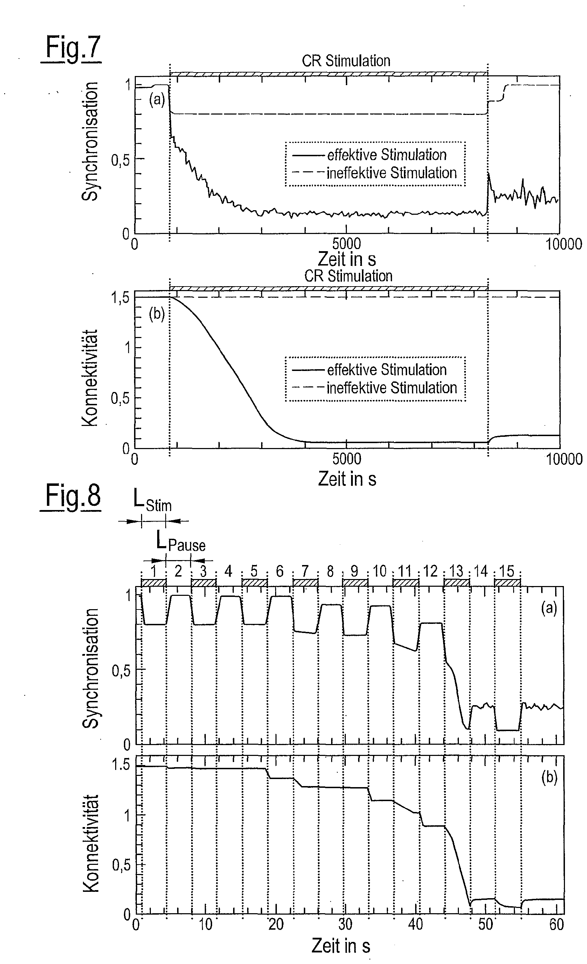

In den Fig. 7 bis 13 werden die mit der hierin beschriebenen Erfindung erzielbaren Effekte anhand von Simulationsergebnissen veranschaulicht.

In den Fig. 7(a) und 7(b) sind der Grad der Synchronisation und die synaptische Konnektivität einer Neuronenpopulation mit einer krankhaft synchronen und oszillatorischen neuronalen Aktivität vor, während und nach einer CR-Stimulation dargestellt. Die in den beiden Darstellungen oben eingezeichneten horizontalen Balken geben den Zeitraum an, in dem die CR-Stimulation angewendet wird.

Wie die Fig. 7(a) und 7(b) zeigen, bewirkt eine effektive CR-Stimulation eine rasche Desynchronisation der Neuronenpopulation sowie eine starke Verringerung der Konnektivität. Jedoch kann sich unter bestimmten Umständen ein nur geringer Stimulationserfolg einstellen, was sich daran ablesen lässt, dass sich der Synchronisationsgrad und die Konnektivität innerhalb der stimulierten Neuronenpopulation trotz der CR-Stimulation nur geringfügig verringern.

Mit den oben beschriebenen Vorrichtungen 1 und 2 lässt sich die Effizienz der CR-Stimulation durch die Einfügung von Stimulationspausen bei geringen Reizstärken verbessern. Weiterhin können z. B. im Fall von ungünstig platzierten Elektroden und/oder Nebenwirkungen Stimulationspausen eingefügt werden, um eine effiziente Stimulation bei geringen Reizstärken zu ermöglichen.

Eine ungünstig platzierte Elektrode ist eine Elektrode, bei der zu viele ihrer Stimulationskontakte ineffektiv sind. Insbesondere wird eine Elektrode dann als ungünstig platziert bewertet, wenn so viele Stimulationskontakte der Elektrode ineffektiv sind, dass nicht mehr mindestens 3 effektive Stimulationskontakte vorhanden sind. Ein Stimulationskontakt ist ineffizient, wenn eine Reizung mit einem Ensemble von Einzelreizen über den betreffenden Stimulationskontakt nicht zu einer Phasenrücksetzung der krankhaften synchronen Oszillation (im Vergleich zur prästimulus-Baseline) führt bzw. bei dem eine periodische Reizung nicht zu einer hinreichend starken n:m-Phasensynchronisation zwischen periodischem Reiz und synchroner Oszillation führt.

Nebenwirkungen hängen von der jeweiligen Erkrankung und dem jeweils gewählten Zielgebiet ab. Es können z. B. als Nebenwirkung von displatzierten Elektroden Dyskinesien auftreten, die sich durch eine Koaktivierung (anstatt einer alternierenden Aktivierung) von antagonistischen Muskeln (z. B. Beugern und Streckern) zeigen. Nebenwirkungen können sich auch zeigen durch eine stimulationsabhängige Zunahme von synchroner Aktivität in entsprechenden Sensoren.

Fig. 8(a) und 8(b) zeigen die Ergebnisse einer Stimulation, die alternierende Stimulationsphasen, in denen eine CR-Stimulation durchgeführt wird, und Stimulationspausen, in denen keine Stimulation durchgeführt wird, umfasst. Die Stimulationsphasen sind in den Fig. 8(a) und 8(b) durch horizontale Balken gekennzeichnet. Die Längen L_{Stim} und L_{Pause} der Stimulationsphasen und -pausen sind in dem vorliegenden Beispiel gleich lang und betragen jeweils 3.600 s. Bis auf die Stimulationspausen wurden für die in den Fig. 8(a) und 8(b) dargestellten Simulationen die gleichen Stimulationsparameter verwendet wie für die in den Fig. 7(a) und 7(b) gezeigte Simulation der ineffektiven Stimulation. Das Einschieben der Stimulationspausen führt zu einer deutlichen Abnahme des Synchronisationsgrads und der Konnektivität. Das Einschieben der Stimulationspausen bewirkt folglich, dass eine ansonsten wirkungslose Stimulation effektiv ist. Ferner lassen sich mit dieser Stimulationsform lang anhaltende therapeutische Effekte erzielen. Auch nach dem kompletten Ausschalten der Stimulation verbleiben der Synchronisationsgrad und die Konnektivität auf einem sehr geringen Niveau.

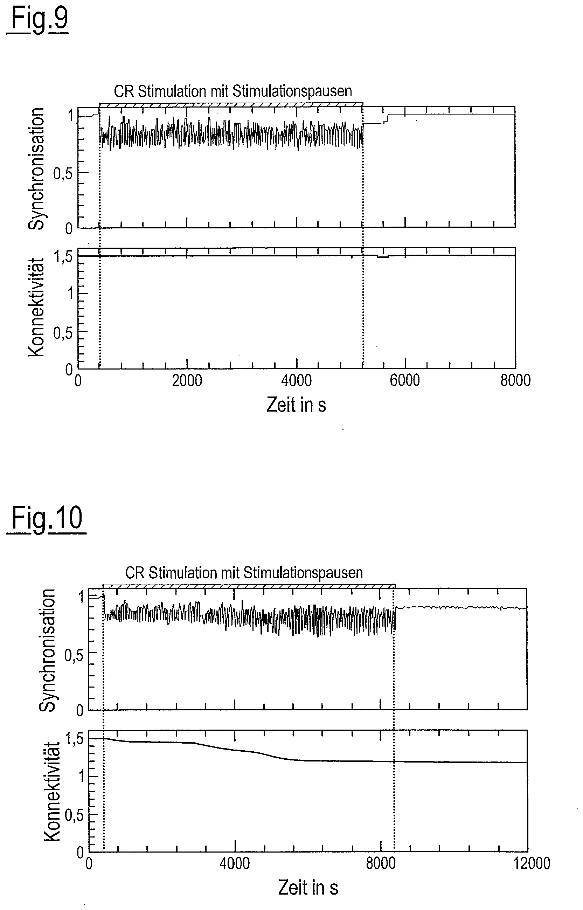

Für den Erfolg der erfindungsgemäßen Stimulationsform ist es wichtig, geeignete Längen L_{Stim} und L_{Pause} für die Stimulationsphasen und -pausen zu ermitteln. Die Fig. 9 bis 12 zeigen die Ergebnisse verschiedener Simulationen, für die bei ansonsten gleichen Stimulationsparametern unterschiedliche Werte für L_{Stim} und L_{Pause} verwendet wurden. Diese Werte sind in der nachfolgenden Tabelle aufgeführt.

| | L_{Stim} | L_{Pause} |
|---|---|---|
| Fig. 9 | 24 s | 24 s |
| Fig. 10 | 24 s | 56 s |
| Fig. 11 | 24 s | 80 s |
| Fig. 12 | 960 s | 720 s |

In den Fig. 9 bis 12 sind durch die horizontalen Balken die Zeiträume gekennzeichnet, in denen die erfindungsgemäße CR-Stimulation mit einander abwechselnden Stimulationsphasen und -pausen durchgeführt wird.

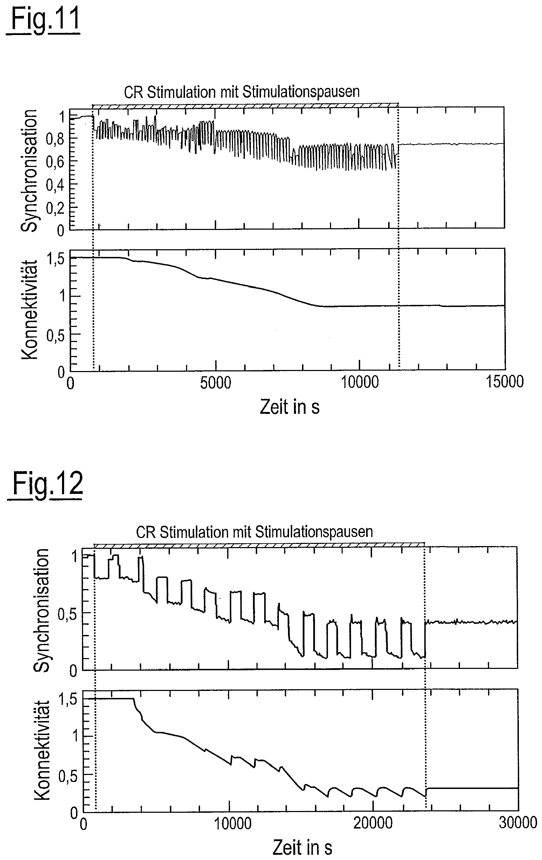

Für nur sehr kurze Stimulationsphasen und -pausen stellt sich ein nur sehr geringer Effekt ein (vgl. Fig. 9), der darüber hinaus nach dem Beenden des Stimulationsvorgangs von nur kurzer Dauer ist. Bessere Ergebnisse werden erzielt, wenn bei gleichbleibender Länge L_{Stim} der Stimulationsphasen die Länge L_{Pause} der Stimulationspausen vergrößert wird (vgl. Fig. 10 und 11). Die besten Ergebnisse werden bei den hier dargestellten Simulationen für relativ große Werte von mehreren Minuten für die Längen L_{Stim} und L_{Pause} erzielt (vgl. Fig. 12).

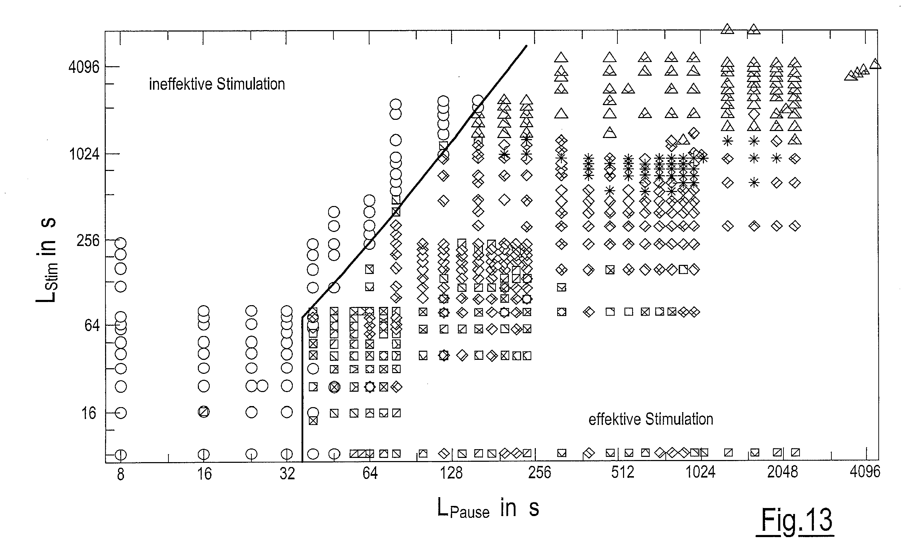

In Fig. 13 sind die Ergebnisse von unterschiedlichen erfindungsgemäßen CR-Stimulationen in einer (L_{Stim}, L_{Pause})-Ebene dargestellt. Die Kreis-Symbole zeigen ineffektive Stimulationen an, alle anderen Symbole stehen für effektive Stimulationen. Ferner ist die (L_{Stim}, L_{Pause})-Ebene in Fig. 12 durch eine Linie in einen Bereich ineffektiver Stimulation und einen Bereich effektiver Stimulation unterteilt. Wie sich Fig. 12 entnehmen lässt, sind Stimulationen mit nur kurzen Werten für L_{Stim} und L_{Pause} sowie Stimulationen, bei denen L_{Stim} im Vergleich zu L_{Pause} zu lang ist, ineffektiv. Die besten Stimulationsergebnisse wurden erzielt für Parameterwerte aus dem oberen rechten Bereich der (L_{Stim}, L_{Pause})-Ebene.

## Patentansprüche

1. Vorrichtung (2) zur Unterdrückung einer krankhaft synchronen und oszillatorischen neuronalen Aktivität, umfassend
- eine in den Körper eines Patienten implantierbare Stimulationseinheit (11) zur Stimulation von Neuronen im Hirn und/oder Rückenmark des Patienten, die eine krankhaft synchrone und oszillatorische neuronale Aktivität aufweisen, mit elektrischen und/oder optischen Reizen (22), wobei die Reize (22) dazu ausgelegt sind, bei einer Verabreichung an den Patienten die krankhaft synchrone und oszillatorische neuronale Aktivität zu unterdrücken,
- eine Messeinheit (12) zum Aufnehmen von Messsignalen (23), die eine neuronale Aktivität der stimulierten Neuronen wiedergeben, und
- eine Steuer- und Analyseeinheit (10) zur Steuerung der Stimulationseinheit (11) und zur Analyse der Messsignale (23), wobei die Steuer- und Analyseeinheit (10) derart ausgestaltet ist, dass sie
- die Stimulationseinheit (11) derart ansteuert, dass die Stimulationseinheit (11) Reize (22) appliziert, und
- anhand der in Reaktion auf die Applikation der Reize (22) aufgenommenen Messsignale (23) den Stimulationserfolg überprüft,
**dadurch gekennzeichnet, dass**
- die Steuer- und Analyseeinheit (10) ferner derart ausgestaltet ist, dass sie, falls der Stimulationserfolg nicht ausreichend ist, eine oder mehrere Stimulationspausen in die Applikation der Reize (22) einfügt oder eine oder mehrere Stimulationspausen verlängert, wobei während der Stimulationspausen keine Reize appliziert werden, welche die krankhaft synchrone und oszillatorische neuronale Aktivität unterdrücken könnten.

2. Vorrichtung (2) nach Anspruch 1, wobei die Steuer- und Analyseeinheit (10) ferner derart ausgestaltet ist, dass sie die Dauer der Stimulationspausen insbesondere schrittweise verlängert, bis die Steuer- und Analyseeinheit (10) feststellt, dass der Stimulationserfolg ausreichend ist.

3. Vorrichtung (2) nach Anspruch 2, wobei die Steuer- und Analyseeinheit (10) ferner derart ausgestaltet ist, dass sie neben der Dauer der Stimulationspausen die Dauer der Stimulationsphasen insbesondere schrittweise verlängert, bis die Steuer- und Analyseeinheit (10) feststellt, dass der Stimulationserfolg ausreichend ist.

4. Vorrichtung (2) nach einem der vorhergehenden Ansprüche, wobei
- die Stimulationsphasen jeweils eine Dauer L_{Stim} und die zwischen aufeinander folgenden Stimulationsphasen eingehaltenen Stimulationspausen jeweils eine Dauer L_{Pause} aufweisen und A = L_{Stim} = L_{Pause} oder A = L_{Stim}/L_{Pause} gilt, und
- die Steuer- und Analyseeinheit (10) ferner derart ausgestaltet ist, dass sie, falls der Stimulationserfolg nicht ausreichend ist, A insbesondere schrittweise vergrößert.

5. Vorrichtung (2) nach Anspruch 4, wobei die Steuer- und Analyseeinheit (10) ferner derart ausgestaltet ist, dass sie, falls der Stimulationserfolg nicht ausreichend ist, A für A = L_{Stim}/L_{Pause} von 1/n auf n insbesondere schrittweise vergrößert, wobei n insbesondere eine Zahl im Bereich von 2 bis 10 ist.

6. Vorrichtung (2) nach Anspruch 4 oder 5, wobei die Steuer- und Analyseeinheit (10) ferner derart ausgestaltet ist, dass sie A so lange konstant hält, bis die Steuer- und Analyseeinheit (10) feststellt, dass der Stimulationserfolg nicht ausreichend ist.

7. Vorrichtung (2) nach einem der Ansprüche 4 bis 6, wobei die Steuer- und Analyseeinheit (10) ferner derart ausgestaltet ist, dass sie A bei nicht ausreichendem Stimulationserfolg so lange vergrößert, bis die Steuer- und Analyseeinheit (10) feststellt, dass der Stimulationserfolg ausreichend ist oder ein Abbruchkriterium erfüllt ist.

8. Vorrichtung (2) nach einem der vorhergehenden Ansprüche, wobei die Dauer einer Stimulationspause mindestens 3 Minuten beträgt.

9. Vorrichtung (2) nach einem der vorhergehenden Ansprüche, wobei die Dauer einer Stimulationspause der Dauer von mindestens 200 oder mindestens 1.000 Perioden der krankhaft synchronen und oszillatorischen neuronalen Aktivität entspricht.

## Claims

1. An apparatus (2) for suppressing an abnormally synchronous and oscillatory neuronal activity, the apparatus comprising
- a stimulation unit (11) that is implantable into a patient's body for stimulating with electric and/or optical stimuli (22) neurons in the patient's brain and/or spinal cord which comprise an abnormally synchronous and oscillatory neuronal activity, wherein the stimuli (22) are configured to suppress the abnormally synchronous and oscillatory neuronal activity when being applied to the patient,
- a measuring unit (12) for recording measuring signals (23) which reproduce a neuronal activity of the stimulated neurons, and
- a control and analysis unit (10) for controlling the stimulation unit (11) and analyzing the measuring signals (23), wherein the control and analysis unit (10) is configured such that it
- controls the stimulation unit (11) such that the stimulation unit (11) applies the stimuli (22), and
- verifies the stimulation success with the help of the measuring signals (22) recorded in reaction to the applied stimuli (22),
**characterized in that**
- the control and analysis unit (10) is further configured such that, if the stimulation success is insufficient, it inserts one or more stimulation breaks during application of the stimuli (22) or extends one or more stimulation breaks, wherein during the stimulation breaks no stimuli are applied that could suppress the abnormally synchronous and oscillatory neuronal activity.

2. The apparatus (2) according to claim 1, wherein the control and analysis unit (10) is further configured such that it extends the duration of the stimulation breaks, particularly step-by-step, until the control and analysis unit (10) determines that the stimulation success is sufficient.

3. The apparatus (2) according to claim 2, wherein the control and analysis unit (10) is further configured such that, apart from the duration of the stimulation breaks, it extends the duration of the stimulation phases, particularly step-by-step, until the control and analysis unit (10) determines that the stimulation success is sufficient.

4. The apparatus (2) according to any one of the preceding claims, wherein
- each of the stimulation phases comprises a duration L_{Stim} and each of the stimulation breaks observed between successive stimulation phases comprises a duration L_{Pause}, and A = L_{Stim} = L_{Pause} or A = L_{Stim}/L_{Pause} applies, and
- the control and analysis unit (10) is further configured such that, if the stimulation success is insufficient, it increases A, particularly step-by-step.

5. The apparatus (2) according to claim 4, wherein the control and analysis unit (10) is further configured such that, if the stimulation success is insufficient, it increases A for A = L_{Stim}/L_{Pause} from 1/n to n, particularly step-by-step, wherein n is particularly a number in the range of 2 to 10.

6. The apparatus (2) according to claim 4 or 5, wherein the control and analysis unit (10) is further configured such that it will keep A constant until the control and analysis unit (10) determines that the stimulation success is insufficient.

7. The apparatus (2) according to any one of claims 4 to 6, wherein the control and analysis unit (10) is further configured such that, when the stimulation success is insufficient, it will increase A until the control and analysis unit (10) determines that the stimulation success is sufficient or a termination criterion is fulfilled.

8. The apparatus (2) according to any one of the preceding claims, wherein the duration of a stimulation break is at least 3 minutes.

9. The apparatus (2) according to any one of the preceding claims, wherein the duration of a stimulation break corresponds to the duration of at least 200 or at least 1,000 periods of the abnormally synchronous and oscillatory neuronal activity.

## Revendications

1. Dispositif (2) pour réprimer une activité neuronale synchrone et oscillatoire pathologique, comprenant
- une unité de stimulation (11) implantable dans le corps d'un patient pour la stimulation de neurones dans le cerveau et/ou la moelle épinière du patient qui présentent une activité neuronale synchrone et oscillatoire pathologique, avec des excitations électriques et/ou optiques (22), où les excitations (22) sont prévues pour réprimer l'activité neuronale synchrone et oscillatoire pathologique lors d'une administration au patient,
- une unité de mesure (12) pour recevoir des signaux de mesure (23) qui traduisent une activité neuronale des neurones stimulés, et
- une unité de commande et d'analyse (10) pour commander l'unité de stimulation (11) et pour analyser les signaux de mesure (23), où l'unité de commande et d'analyse (10) est agencée de telle manière qu'elle
- commande l'unité de stimulation (11) de telle manière que l'unité de stimulation (11) applique des excitations (22), et
- à l'aide des signaux de mesure (23) reçus en réaction à l'application des excitations (22) vérifie le succès de la stimulation,
**caractérisé en ce que**
- l'unité de commande et d'analyse (10) est en outre agencée de telle manière que, si le succès de la stimulation n'est pas suffisant, elle introduit une ou plusieurs pauses de stimulation dans l'application des excitations (22) ou prolonge une ou plusieurs pauses de stimulation, où pendant les pauses de stimulation aucune excitation qui pourrait réprimer l'activité neuronale synchrone et oscillatoire pathologique n'est appliquée.

2. Dispositif (2) selon la revendication 1, où l'unité de commande et d'analyse (10) est en outre agencée de telle manière qu'elle prolonge en particulier par pas la durée des pauses de stimulation jusqu'à ce que l'unité de commande et d'analyse (10) établisse que le succès de la stimulation est suffisant.

3. Dispositif (2) selon la revendication 2, où l'unité de commande et d'analyse (10) est en outre agencée de telle manière qu'elle prolonge en particulier par pas, outre la durée des pauses de stimulation, la durée des phases de stimulation, jusqu'à ce que l'unité de commande et d'analyse (10) établisse que le succès de la stimulation est suffisant.

4. Dispositif (2) selon l'une des revendications précédentes, où
- les phases de stimulation présentent chacune une durée L_{Stim} et les pauses de stimulation respectées entre les phases de stimulation qui se suivent présentent chacune une durée L_{Pause} et A = L_{Stim} = L_{Pause} ou A = L_{Stim}/L_{Pause}, et
- l'unité de commande et d'analyse (10) est en outre agencée de telle manière que, si le succès de la stimulation n'est pas suffisant, A est augmenté en particulier par pas.

5. Dispositif (2) selon la revendication 4, où l'unité de commande et d'analyse (10) est en outre agencée de telle manière que, si le succès de la stimulation n'est pas suffisant, A pour A = L_{Stim}/L_{Pause} est augmenté en particulier par pas de 1/n à n, où n est en particulier un nombre dans le domaine de 2 à 10.

6. Dispositif (2) selon la revendication 4 ou 5, où l'unité de commande et d'analyse (10) est en outre agencée de telle manière qu'elle maintient A constant jusqu'à ce que l'unité de commande et d'analyse (10) établisse que le succès de la stimulation n'est pas suffisant.

7. Dispositif (2) selon l'une des revendications 4 à 6, où l'unité de commande et d'analyse (10) est en outre agencée de telle manière que, si le succès de la stimulation n'est pas suffisant, elle augmente A jusqu'à ce que l'unité de commande et d'analyse (10) établisse que le succès de la stimulation est suffisant ou qu'un critère d'interruption est respecté.

8. Dispositif (2) selon l'une des revendications précédentes, où la durée d'une pause de stimulation est d'au moins 3 minutes.

9. Dispositif (2) selon l'une des revendications précédentes, où la durée d'une pause de stimulation correspond à la durée d'au moins 200 ou d'au moins 1000 périodes de l'activité neuronale synchrone et oscillatoire pathologique.
